# EUROPEAN PATENT SPECIFICATION

(11) **EP 2 179 755 B1**
(45) Date of publication and mention of the grant of the patent: **13.03.2013**
(21) Application number: 08167548.0
(22) Date of filing: 24.10.2008
(51) Int. Cl.: A61M 5/148, A61J 1/10, A61J 1/14

(54) **Flexible medicine reservoir with an internal reservoir port**
Flexibler Medizinbehälter mit einem internen Behälteranschluss
Réservoir de médicaments flexible doté d'un orifice de réservoir interne

(43) Date of publication of application: 28.04.2010
(73) Proprietor: F.Hoffmann-La Roche AG, 4070 Basel (CH); Roche Diagnostics GmbH, 68305 Mannheim (DE)
(72) Inventor: Wyss, Martin, 3400, Burgdorf (CH)
(74) Representative: Rentsch Partner AG

(56) References cited:
- US-A- 4 632 673
- US-A1- 2004 001 655

## Description

### Field of the Invention

The invention relates to a flexible container for storing a liquid medicament to be administered to a patient by an infusion, especially a therapeutic or diagnostic liquid, and a device for the automated release of a liquid medicament comprising and/or incorporating such a flexible container, according to the preambles of claims 1 and 8.

### State of the art

Devices for the automated release of liquid medicaments are normally used with patients who have a continuous and in the course of the day varying need of a medicine which can be administered by subcutaneous infusion. Specific applications are, for example, certain pain therapies and the treatment of diabetes, in which computer controlled infusion pump devices, such as insulin pumps, are used. Such devices can be carried by a patient on the body, and contain a certain amount of liquid medicament in a medicine reservoir in the form of a container. The medicine reservoir often comprises medicine sufficient for one or several days. The liquid medicament is supplied to the patient's body from the medicine reservoir through an infusion cannula or an injection needle.

Particularly in self-administration of medicaments, for example insulin, the patients using the medicament in question and administering it themselves by means of an infusion pump are increasingly placing importance on convenience and discretion. As a consequence the dimensions of such infusion devices are limited, and particular the overall length should be as small as possible, in order not be evident through clothing and to be carried as comfortable as possible.

Typically said devices are single-use devices for sterility and contamination prevention reasons. Alternatively said devices or parts of it can be reused, for example by replacing an empty medicament container, or by refilling said container by the user. The refilling of containers has the advantage that also medicaments that are not readily available in prefilled containers can be used for such infusion pump devices, thereby providing the patient with a larger choice of sources for his medicaments.

The standard infusion pump devices that are carried on or near the body have a medicine reservoir with a cylindrical ampoule and a displacement piston, which is pushed into the ampoule by a piston rod or threaded spindle in order to convey the liquid medicament. These known designs have the disadvantage of having an overall length that is longer and/or thicker than that desired and which is detrimental to the provision of compact infusion pumps.

Manufacturers try to meet the demand of small infusion pump devices by various means. For example the infusion pump can be divided into structural assemblies which are each arranged in their own, smaller, housings and can joined to one another by wireless or wired connection. An example of such a modular infusion pump device is disclosed in US 2006/0184119 A1.

Another possibility is the use of containers of particularly flat construction. For example may the cylindrical ampoule be replaced by a container with a rectangular or another suitable cross-section, interacting with a displacement piston of corresponding shape. Different embodiments of such compact medicine reservoir devices are shown in WO 2008/122135 A1.

A further approach to reduce the overall volume of an infusion device is to replace the syringe-type dosing mechanism, in which a piston is displaced along a long container axis by an actuator, thereby conveying the appropriate amount of liquid medicine, by a downstream pump system. In such a device a miniaturized pump is arranged downstream of the reservoir, and causes a negative pressure gradient that conveys the product from the reservoir to its destination. An example for such a pump is WO 2004/009162 A1.

For some of such infusion devices, the pressure gradient achievable with such a pump system is not very high. A suitable container for such devices is disclosed in US 2007/0123820 A1, comprising a flat container and a flat piston body arranged in the body in a sliding manner. Fully filled, such a container has a ratio between its maximum height and its overall width of less than 1.25. The cross-section area of the container in relation to the displacement axis is much larger than for conventional cylinder-piston arrangements, and already a comparably small pressure gradient as generated by a miniaturized pump is able to overcome the friction force of the piston sealing gliding on the inner container wall.

In an especially advantageous approach the rigid container and movable piston are replaced by a flexible container. Such a flexible container may for example have the form of two flexible wall sheets that are sealed together. Flexible containers have the advantage of a smaller volume surplus of the container in relation to its content, which reduces the manufacture costs and the achievable dimensions of an infusion pump device with such a flexible container. The volume of a flexible container for use in a infusion pump device may be up to 10 ml, but is preferably 5 ml or less, and more preferably 1.5 to 3.5 ml.

A possible embodiment of an infusion pump device with a flexible container is disclosed in US 2007/0049865 A1. The flexible container comprises a front and rear wall sheet sealed together, and a port that is centrally arranged on one of the wall sheets. The port comprises a flange sealed onto the sheet material, and a septum. A hollow needle penetrates the septum, thereby connecting the container with the infusion cannula. The flexible container containing the liquid medicament is arranged in a second, rigid, fluid-filled container in fluid communication with a primary reservoir of a hydraulic drive fluid through a conduit comprising a flow restrictor. The primary reservoir is in the form of a cartridge with a moveable piston. A spring is arranged to act on the piston, in order to drive fluid from the reservoir to the second container, thereby expelling the medicament from the flexible container when the latter is connected to an infusion needle. The flow rate is determined by the pressure generated in the drive fluid, the viscosity of the drive fluid and the flow resistance in the flow restrictor. Since there is no possibility to easily refill the container, the device is intended for single use. Due to the hydraulic fluid piston and the second, rigid container the total volume of the device is increased. Furthermore the construction is complicated, and the dosing accuracy is limited, due to the indirect pumping method. Such an infusion pump device thus is not very suitable for dosing-critical applications such as the administration on insulin to a diabetes patient.

A common problem of flexible containers with ports as used, for example, in IV bags, is the dead volume resulting between the collapsed container and the port. Said dead volume cannot be used, meaning that it cannot be emptied. Thus a complete draining of the contents of a flexible container is not possible. While said dead volumes are of minor relevance for larger flexible containers as they are used for example for blood preservations, nutritional fluids, or intravenous infusions, containing 100 ml of liquid or more, the resulting loss of useable container volume is considerably higher for smaller containers as they are suitable for infusion pumps, with a total volume of only 5 ml or less. In addition the concentration of the medicament may be much higher than in an intravenous solution container, which further increases the negative effect of the dead volume. In addition to the increased costs, the dead volume leads also to an increase of the overall volume of the flexible container, and thus of the infusion pump device with such a flexible container.

For single-use containers filled with the medicament, the dead volume increases the effective costs per dose and thus of the overall therapy costs, since a certain percentage of the medicament will inevitably remain in the container and has to be disposed. This cost effect is of course especially important for expensive medicaments.

If a container is provided empty, intended to be filled with the appropriate medicament by the user himself, the dead volume is initially filled with air. If said air remains in the container it may lead to dosing errors, which can be dangerous and thus should be avoided. Furthermore the administration of air into a patients body should be avoided in some applications for medical reasons. However, removing the air from the container will require a certain skill of a user. Another problem of flexible containers is the possibility that the container does not collapse homogeneously, the inner sides of the two wall sheets being pressed together due to the pressure difference, and thereby cutting off parts of the container from the port. Such constrictions are particularly problematic for very flexible materials with low elasticity. Other factors influencing the collapsing behaviour of flexible containers are the shape and the size of a container, and the position of the port on the container.

US 2004/0001655 A1 shows a container closure device for the withdrawal of a liquid from a collapsible container, comprising a needle guide attached to the outer surface of the container, and a septum retainer attached to the inner surface of the container. The needle guide and the septum retainer are aligned to each other. Fluid communication between a fluid delivery device and the container is established by piercing a hollow needle through the laminate of the container and the septum.

### Objects of the Invention

One object of the invention is to provide a flexible container for storing a liquid medicament to be administered to a patient, which does not comprise the disadvantages of the known containers. Particularly a flexible container according to the invention should have a reduced dead volume.

Another object of the invention is to provide an advantageous port for such flexible containers.

A further object of the invention is to provide a device for the automated release of a liquid medicament comprising and /or incorporating such a flexible container.

These and other objects are achieved by a flexible container and a device for the automated release of a liquid medicament according to claims 1 and 8. Advantageous embodiments are given in the dependent claims.

### Summary of the invention

A flexible container for storing a liquid medicament according to the invention comprises two walls consisting of a flexible, sheet-like material, and at least one port mounted to a wall. The at least one port has a flange that is sealingly attached to the wall. Liquid, particularly the liquid medicament to be administered to a patient with an infusion pump device, is transferred to and from the container through the port. An inner conduit of the port connects the inner storage volume of the container and the exterior of the container. The port may also be used to deaerate the container.

The at least one port comprises a base plate facing toward the interior of the container and an adapter protruding through a hole in a wall of the flexible container. One or more drain channels are arranged on the base plate, and are connected to the inner conduit of the port via an inner opening located on the base plate. Due to the drain channels remaining fluid can flow to the inner conduit even if the container is collapsed, and the base plate is firmly abutting the opposite wall. Preferably the base plate comprises a network of interconnected drain channels.

In an advantageous embodiment the flange of the at least one port is arranged in a depression on the inner side of the wall, and preferably the shape of the base plate is such that the base plate and the adjacent area of the inner side of the wall form an essentially flat and smooth surface.

In another advantageous embodiment the port comprises further functional elements, such as for example a bubble filter, arranged in the inner conduit, a pressure sensor, a pressure transfer membrane for coupling the inner conduit to a pressure sensor. It may also comprise a pumping mechanism, or part of a pumping mechanism, such as a micro membrane pump, or a micro plunger pump.

As used herein, the terms "medicament" and "liquid medicament" are meant to encompass any drug-containing flowable medicine, or therapeutic or diagnostic liquid, capable of being passed through a delivery means such as a hollow needle in a controlled manner, such as a liquid, solution, gel or fine suspension. Representative drugs include pharmaceuticals such as peptides, proteins, and hormones, biologically derived or active agents, hormonal and gene based agents, nutritional formulas and other substances in both solid (dispensed) or liquid form. In particular the term medicament encompasses insulin preparations ready for administration.

The terms "subcutaneous infusion" and "subcutaneous injection" are meant to encompass any method in which a needle device is inserted at a selected site within the body of a patient for subcutaneous, intravenous, intramuscular or intradermal delivery of a liquid medicament to a subject. Further, the term needle defines a piercing member (including an array of micro needles) adapted to be introduced into or through the skin of a subject.

The terms "drain channel" and "drain channel network" are meant to encompass any arrangement of depressions and protrusions on a surface that provide sufficient interconnected space between the surface and a flexible sheet firmly abutted to said surface that a fluid can flow through said space.

### Brief description of the drawings

In order to facilitate a fuller understanding of the present invention, reference is now made to the appended drawings. These references should not be construed as limiting the present invention, but are intended to be exemplary only.
- Figure 1: schematically shows a possible embodiment of a flexible container according to the invention, (a) in a perspective view, (b) in a cross-section, and (c) in an enlarged detail view A of the cross-section of the port.
- Figure 2: schematically shows a port as shown in Figure 1, (a) in a cross-sectional view, (b) in a perspective view onto the base plate, and (c) in a perspective onto the flange and the adapter.
- Figure 3: schematically shows four different variants for drain channel arrangements of ports , in a perspective view (a), (b) and with view onto the base plate (c), (d).
- Figure 4: schematically shows a cross-section of a further embodiment of a flexible container according to the invention, (a) in a completely empty state, and (b) in a fully filled state.
- Figure 5: shows a schematical cross-section of two other embodiments of a flexible container according to the invention.
- Figure 6: shows a schematical cross-section of yet two other embodiments of a flexible container according to the invention.
- Figure 7: shows a top view on another embodiment of a flexible container.
- Figure 8: shows a schematical cross-section of an embodiment of a flexible container according to the invention with two ports, sharing a common base plate.

### Description of embodiments of the invention

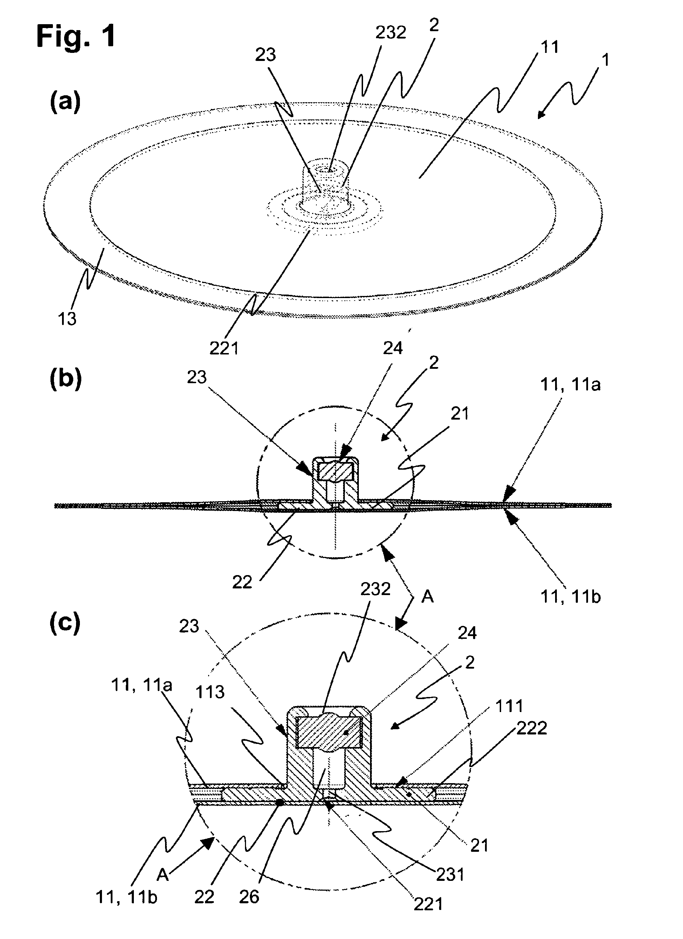

An embodiment of a flexible container according to the invention is shown in Figure 1. The flexible container 1 is shown in the empty state, and comprises two walls 11 and a port 2, arranged in the centre of one of the essentially circularly shaped walls. The walls 11 consists of two sheets 11 a, 11 b of flexible, liquid-tight material sealed along a circumferential sealing rim 13. The port 2 comprises a flange 21 for mounting the port on the wall 11, an adapter 23 for connecting the port to an infusion pump device, or to a device for filling or refilling the container, and a base plate 22 facing toward the inner storage volume of the flexible container. Base plate 22 is formed by flange 21 and the longitudinal end of adapter 23 facing toward the inner storage volume. An inner conduit 26 leads from an outer opening 232 to an inner opening 231 arranged in the centre of the base plate 22. In the shown embodiment a septum 24, for example made from silicon polymer, is arranged in the inner conduit 26. This particular embodiment is thus suitable for the use of a hollow needle penetrating the septum 24 to connect the container to an outer conduit system, and to transfer a liquid medicament into and out of the flexible container 1. The flange 21 of the port is connected in a liquid tight manner to the inner side 111 of the wall, for example by ultrasonic welding. The adapter 23 protrudes through a hole 113 in the wall of the container.

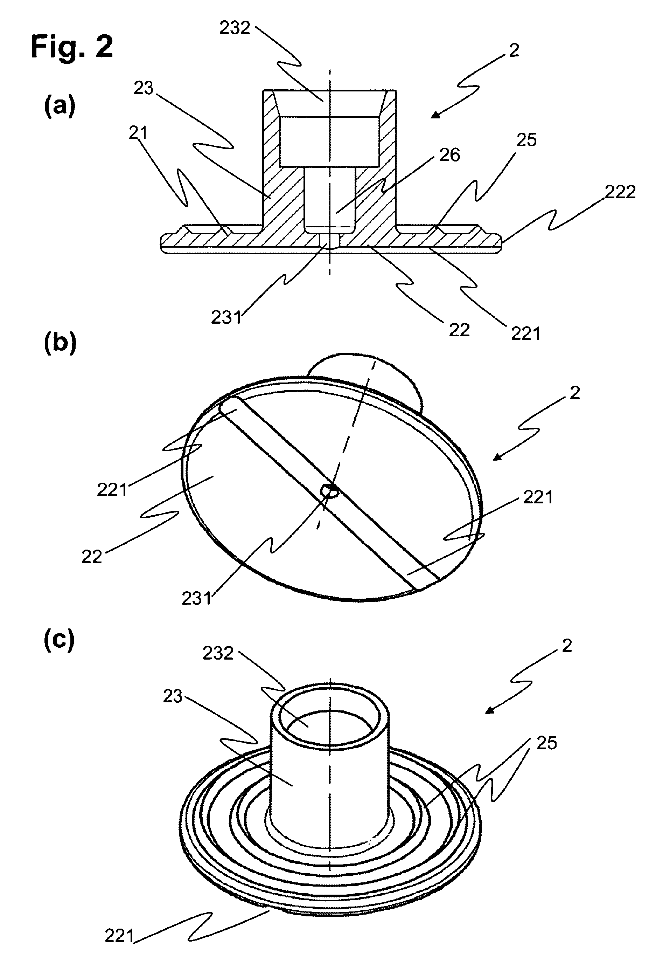

The port 2 according to the invention is shown in more detail in Figure 2. According to the invention a drain channel 221 is arranged across the diameter of the base plate, the inner opening 231 opening out into the drain channel 221. Since the drain channel is parallel to the viewing axis of Figure 1(a) and (b), it is only visible in its cross-section. However, it is clearly visible in the perspective view shown in Figure 2(b) along the axis of the drain channel 221.

In the port in Figure 2 the septum 24 is not yet mounted. After manufacturing the port, for example by injection moulding, the septum is placed in the broader upper area of the inner conduit 26, and the upper edge of the adapter are thermally deformed inwardly to fixate the septum. In Figure 2(a) and (c) energy directors 25 for ultrasonic welding are shown, which are circumferentially arranged on the flange 21.

When the liquid content of the flexible container 1 is removed during the use of an infusion pump device, the walls 11 of the container collapse as a result of the pressure difference between the inner storage volume and the exterior pressure. While the container is emptied, the two sheets 11 a, 11 b of the wall touch each other. Touching of the two sheets 11a, 11b of the wall may start from the sealing rim 13, but may also start at a different location, dependent on factors such as the elasticity of the wall 11 and the orientation with respect to gravity. When the base plate 22 finally touches the opposing sheet 11b, there still remains a certain distance between the sheets 11 a, 11 b in the area adjacent to the edge 22 of the base plate, since the base plate has a certain height. The content of this last remaining volume may then flow through the drain channel 221 into the inner conduit 26, until the container 1 is completely drained and collapsed. Vice versa, if liquid content is pumped into a completely empty container, the liquid is distributed via the drain channel, and the two sheets sticking to each other are separated in a controlled manner.

In an advantageous embodiment a circumferential channel is arranged along the edge of the base plate, facing toward the sealing rim. This will ensure an ideal connection between the surrounding remaining container volume and even one single drain channel.

The sealing of the sheets forming the wall may be achieved by heat sealing, ultrasonic welding, high-frequency inductive welding, glueing, or any other suitable method for producing flexible containers from sheet-like material that is known to the skilled person. Similar methods may be used for attaching the flange of the port to the container wall. The sheet-like material may be a single layer foil of a suitable polymer, or a multi layer structure foil. The base area of a flexible container according to the invention may have any suitable shape, particularly square, rectangular, circular, oval, hexagonal shape. The shape may also be especially adapted to a specific infusion pump device. Instead of sealing together two separate sheets, the walls of the container may also be produced from a single sheet that is folded along an axis, and is sealed along the remaining edges. Another possibility known from the state of the art is the use of continuous film tubes for producing the containers.

The sheet like material of the wall can be a monolayer film or a multilayer structure. The container wall may consist of one or more polymers of the following families: Polypropylene (PP), Polyethylene (PE), and copolymers; Ethylene Vinyl Acetate (EVA), Polyvinyl Chloride (PVC), Polyvinylidene Chloride (PVDC), Polystyrene (PS), Ethylene Vinyl Alcohol (EVOH), Polyethylene Terephthalate (PET), Polyamide (PA), Polychlorotrifluoroethylene (PCTFE), Cyclic Olefin Copolymer (COC), Thermoplastic Elastomer (TPE), or generally any other polymer material which is known to the skilled person to be suitable for that purpose.

The wall may be manufactured for example by extrusion, blown film extrusion, coextrusion or lamination. When producing a multilayer structure it may be necessary to include one or more tie layers, or to apply one or more adhesive layers between the functional layers. To improve barrier properties one may also use metallised film, or a silicon oxide or aluminium oxide coating may be applied.

The port may consist of a polymer of the following families: Polypropylene (PP), Polyethylene (PE), and copolymers; Ethylene Vinyl Acetate (EVA), Polyvinyl Chloride (PVC), or generally any other polymer material which is known to the skilled person to be suitable for that purpose.

Depending on the used process to attach the port to the wall of the container it may be advantageous if the port and the adjacent layer of the wall are made of materials of the same family.

Instead of being attached to the inner side of the wall of the container, the flange of the port may of course also be attached to the outer side of the wall. In such a case only that part of the base plate not being part of the flange will face the inner volume of the container. This variant has the advantage that the port can be mounted in a simpler manner, since the adapter of the port does not have to be positioned in a hole of the wall, but may be simply placed on said hole. Furthermore the detrimental dead volume is even more reduced, and allows the container to be fully flat when emptied.

The first variant with the flange lying inside, however, has the advantage that the container is more secure, since the inner pressure will press the flange against the wall, thereby reinforcing the sealing. In addition the first variant is advantageous when the sheet material is a compound foil, since there is no contact between the outer foil layers and the content of the container.

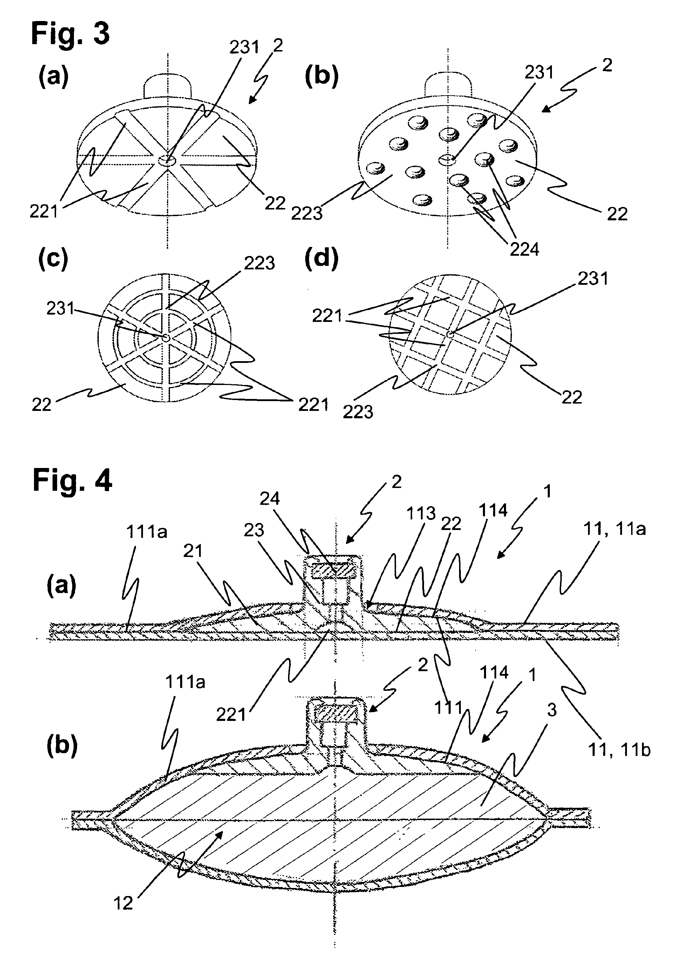

Examples of other possible arrangements of drain channels 221 of ports 2 are given in Figure 3. Figure 3(a) shows a port 2 with a base plate 22 on which three drain channels 221 are arranged in a star-like manner. In the variant in Figure 3(b) a number of protrusions 224 are arranged on the base plate 22, the interstice between said protrusions 224 forming a network 223 of drain channels 221. In Figure 3(c) three linear and two circular drain channels 221 are arranged on the base plate, while in Figure 3(d) the drain channels 221 form a grid-like network 223.

Generally the drain channels should be as shallow as possible, in order to decrease the dead volume. Among other factors, the achievable minimum depth of the drain channels of a given channel network in order to avoid blocking of the channels depends on the flexibility of the sheet material and the width of the channels. Furthermore a given minimum flow must be ensured, which depends for example on the demands of the dosing pump and the viscosity of the liquid medicament.

Another advantageous embodiment of a flexible container 1 according to the invention is shown in Figure 4. As in Figure 1 the cross-section is perpendicular to the axis of the single drain channel 221. The flange 21 is convexly shaped, and is arranged in a correspondingly shaped depression 114 on the inner side 111 of the wall sheet 11a. Said depression 114 may be preformed or may result fully or partially from the elasticity of the wall 11. Such an embodiment has the advantage that the dead volume of the container is further decreased, since in the completely drained state, as shown in Figure 4(a), the base plate 22 and the adjacent area 111a of the inner side of the wall 11 form an essentially smooth surface. When the two sheets 11 a, 11 b collapse, no dead volume remains between them.

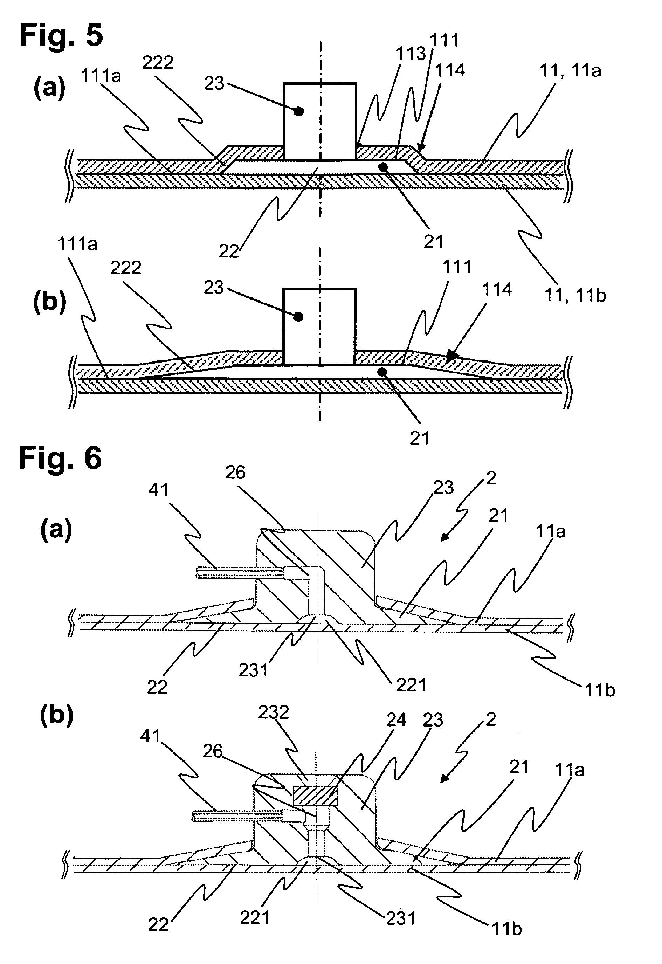

Two other variants of such an embodiment of a flexible container are given in Figure 5. As in Figure 4, the flange 21 is arranged in a depression 114 of the wall 11, the base plate 22 and the adjacent area 111a forming an essentially smooth and flat surface, in the empty state of the container. Said depression 114 may be preformed or may result fully or partially from the elasticity of the wall 11.

Figure 6 shows two embodiments in which an outer conduit system 41, for example of an infusion pump device, is directly connected to the inner conduit 26 of the port 2. The outer conduit is arranged perpendicular to the inner conduit, in order to reduce the overall device volume. In Figure 6(b) the outer conduit 41 is combined with a septum 24. Such a variant is particularly advantageous, since it can be connected to an infusion pump device in a space-saving manner via the outer conduit 41, but may at the same time be filled by a user with a syringe, using the septum 24. In another possible variant, a port may be connected to more than one outer conduit.

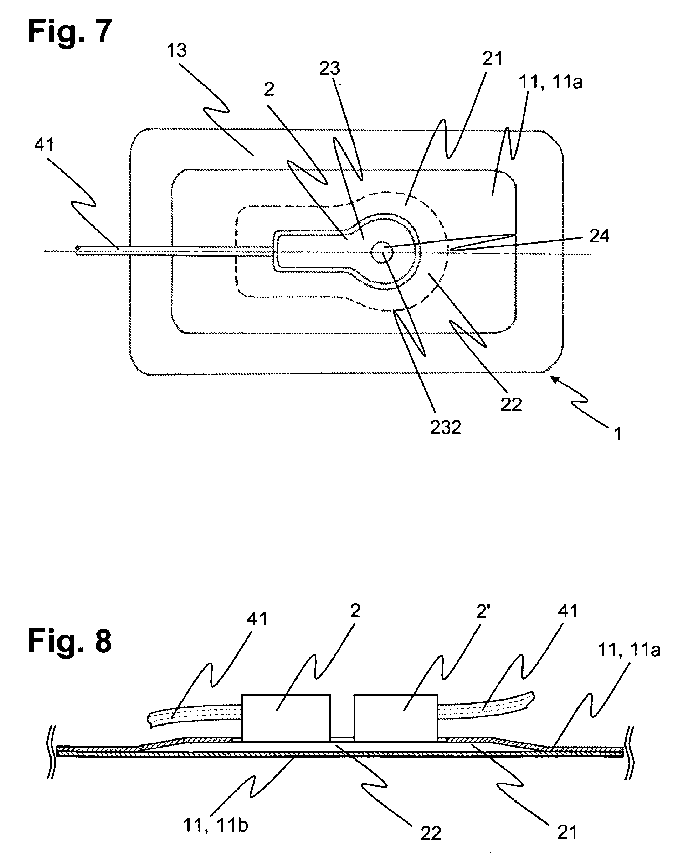

Another embodiment of a flexible container is given in Figure 7, in a top view onto the container and the port. The port 2, with the adapter 23 and the base plate 22, is pear shaped, with an outer conduit 41 leading to the thinner end of the adapter 23, and an outer opening 232 with visible septum 24 on the circular part of the adapter 23.

Of course a port may not only have a circular shape, but may have any suitable regular or irregular shape that provides the intended functionality. Furthermore instead of being arranged in the centre of the container, the port 2 may have any suitable position on the container, for example near the sealing rim 13. It is also possible to arrange the port 2 partially inside the sealing rim 13.

Figure 8 shows an embodiment of a flexible container with two separate ports 2, 2' connected to separate outer conduits 41. In the shown example the two ports share a common base plate 22. However, of course a container according to the invention may also contain two or more fully separate ports. It may also contain additional ports to deaerate the container. In another embodiment of the invention the port of the container according to the invention is an integral part of an infusion pump device.

In one advantageous embodiment of the invention, the drain channel network of the base plate of the port is extended to the walls of the container. This can be achieved for example by hot embossing a grid of lines on at least a part of the inner surface of the wall, particularly on one or both sheets of the wall. In such an embodiment the drain channels of the port are connected to the embossed grid line network. No portion of the content can be blocked and separated from the port when the container collapses in a sub-optimal manner during emptying, even for very large container or very flexible container walls, since the liquid can always flow to the port through the grid line network.

The present invention is not to be limited in scope by the specific embodiments described herein. Indeed, various modifications of the present invention, in addition to those described herein, will be apparent to those skilled in the art from the foregoing description and accompanying drawings.

### List of Reference Numerals

- 1: flexible container
- 11: wall
- 11a, 11b: sheet
- 111: inner side of the wall
- 111a: area adjacent to the base plate
- 113: hole
- 114: depression
- 12: inner storage volume
- 13: sealing rim
- 2, 2': port
- 21: flange
- 22: base plate
- 221: drain channel
- 222: edge
- 223: drain channel network
- 224: protrusion
- 23: adapter
- 231: inner opening
- 232: outer opening
- 24: septum
- 25: energy director
- 26: inner conduit
- 3: liquid medicament
- 41: outer conduit system

## Claims

1. A flexible container (1) for storing a liquid medicament (3), comprising two walls (11, 11a, 11b) consisting of a flexible, sheet-like material, and at least one port (2) mounted to a wall (11, 11a, 11 b) for transferring liquid (3) and/or gas to and/or from an inner storage volume (12) of the container (1), the at least one port (2) having a flange (21) that is sealingly attached to said wall (11, 11 a, 11b), an adapter (23) for connecting the port (2), and a base plate (22) facing toward the inner storage volume (12) and having at least one drain channel (221) arranged on said base plate (22), wherein an inner conduit (26) connects the inner storage volume (12) and the exterior of the container, and the at least one drain channel (221) being connected to the inner conduit (26) via an inner opening (231) located on the base plate (22), **characterised in that** the adapter (23) protrudes through a hole (113) in a wall (11, 11a, 11b).

2. The flexible container according to claim 1, **characterized in that** the at least one drain channel (221) of the base plate (22) stretches from the inner opening (231) of the inner conduit (26) to an outer edge (222) of the base plate (22).

3. The flexible container according to claim 1 or 2, **characterized in that** the base plate (22) comprises a network (223) of interconnected drain channels (221).

4. The flexible container according to one of the preceding claims, **characterized in that** the inner conduit (26) is an integral part of the inner opening (231), particularly that the inner conduit (26) and the inner opening (231) are formed by the same bore or hole.

5. The flexible container according to one of the preceding claims, **characterized in that** the flange (21) of the at least one port (2) is arranged in a depression (114) on the inner side (111) of the wall (11).

6. The flexible container according to one of the preceding claims, **characterized in that** the shape of the base plate (22) is such that the base plate (22) and the adjacent area (111a) of the inner side of the wall (11) form an essentially flat and smooth surface.

7. The flexible container according to one of the preceding claims, **characterized in that** the at least one port (2) comprises a further functional element, for example a bubble filter, a pressure sensor, a pressure transfer membrane, or a pumping mechanism, complete or in part.

8. A device for the automated release of a liquid medicament, particularly an infusion pump device, **characterised by** comprising and/or incorporating at least one flexible container (1) according to any of claims 1 to 7.

## Patentansprüche

1. Flexibler Behälter (1) zum Speichern eines flüssigen Medikaments (3), umfassend zwei Wände (11, 11a, 11b) bestehend aus einem flexiblen, folienförmigen Material, und wenigstens einen an einer Wand (11, 11a, 11b) angebrachten Anschluss (2) zum Übertragen von Flüssigkeit (3) und/oder Gas in ein und/oder von einem inneren Speichervolumen (12) des Behälters (1), wobei der wenigstens eine Anschluss (2) einen Flansch (21) aufweist, der an der genannten Wand (11, 11a, 11b) dichtend befestigt ist, einen Adapter (23) zum Verbinden des Anschlusses (2) und eine Basisplatte (22), die dem inneren Speichervolumen (12) zugewandt ist und wenigstens einen Ablaufkanal (221) aufweist, der auf der genannten Basisplatte (22) angeordnet ist, und wobei eine innere Leitung (26) das innere Speichervolumen (12) und die Aussenseite des Behälters miteinander verbindet, und der wenigstens eine Ablaufkanal (221) über eine innere Öffnung (231), die sich auf der Basisplatte (22) befindet, mit der inneren Leitung (26) verbunden ist, **dadurch gekennzeichnet, dass** der Adapter (23) durch ein Loch (1 13) in einer Wand (11, 11a, 11b) hinausragt.

2. Flexibler Behälter nach Anspruch 1, **dadurch gekennzeichnet, dass** der wenigstens eine Ablaufkanal (221) der Basisplatte (22) sich von der inneren Öffnung (231) der inneren Leitung (26) zu einer äusseren Kante (222) der Basisplatte (22) erstreckt.

3. Flexibler Behälter nach Anspruch 1 oder 2, **dadurch gekennzeichnet, dass** die Basisplatte (22) ein Netzwerk (223) aus miteinander verbundenen Ablaufkanälen (221) umfasst.

4. Flexibler Behälter nach einem der vorangehenden Ansprüche, **dadurch gekennzeichnet, dass** die innere Leitung (26) integraler Bestandteil der inneren Öffnung (231) ist, insbesondere dass die innere Leitung (26) und die innere Öffnung (231) durch dieselbe Bohrung oder dasselbe Loch gebildet sind.

5. Flexibler Behälter nach einem der vorangehenden Ansprüche, **dadurch gekennzeichnet, dass** der Flansch (21) des wenigstens einen Anschlusses (2) in einer Vertiefung (114) auf der Innenseite (111) der Wand (11) angeordnet ist.

6. Flexibler Behälter nach einem der vorangehenden Ansprüche, **dadurch gekennzeichnet, dass** die Gestalt der Basisplatte (22) derart ist, dass die Basisplatte (22) und der benachbarte Bereich (111a) der Innenseite der Wand (11) eine im Wesentlichen flache und glatte Oberfläche bilden.

7. Flexibler Behälter nach einem der vorangehenden Ansprüche, **dadurch gekennzeichnet, dass** der wenigstens eine Anschluss (2) ein weiteres funktionelles Element umfasst, zum Beispiel einen Blasenfilter, einen Drucksensor, eine Druckübertragungsmembran, oder einen Pumpmechanismus, vollständig oder Teile davon.

8. Vorrichtung zur automatische Abgabe eines flüssigen Medikaments, insbesondere eine Infusionspumpenvorrichtung, **dadurch gekennzeichnet, dass** sie wenigstens einen flexiblen Behälter (1) nach irgendeinem der Ansprüche 1 bis 7 umfasst und/oder enthält.

## Revendications

1. Contenant souple (1) pour stocker un médicament liquide (3), comprenant deux parois (11, 11a, 11b) consistant en une matière souple, de type feuille, et au moins un orifice (2) monté sur une paroi (11, 11a, 11b) pour transférer du liquide (3) et/ou du gaz vers et/ou à partir d'un volume de stockage intérieur (1 2) du contenant (1), le au moins un orifice (2) ayant une bride (21) qui est fixée de façon étanche à ladite paroi (11, 11a, 11b), un adaptateur (23) pour une liaison à l'orifice (2), et une plaque de base (22) tournée vers le volume de stockage intérieur (12) et ayant au moins un canal de drainage (221) agencé sur ladite plaque de base (22), un conduit intérieur (26) reliant le volume de stockage intérieur (12) et l'extérieur du contenant, et le au moins un canal de drainage (221) étant relié au conduit intérieur (26) par l'intermédiaire d'une ouverture intérieure (231) située sur la plaque de base (22), **caractérisé par le fait que** l'adaptateur (23) fait saillie à travers un trou (113) dans une paroi (11, 11a, 11b).

2. Contenant souple selon la revendication 1, **caractérisé par le fait que** le au moins un canal de drainage (221) de la plaque de base (22) s'étire de l'ouverture intérieure (231) du conduit intérieur (26) à un bord extérieur (222) de la plaque de base (22).

3. Contenant souple selon l'une des revendications 1 ou 2, **caractérisé par le fait que** la plaque de base (22) comprend un réseau (223) de canaux de drainage interconnectés (221).

4. Contenant souple selon l'une des revendications précédentes, **caractérisé par le fait que** le conduit intérieur (26) est une partie d'un seul tenant de l'ouverture intérieure (231), en particulier **par le fait que** le conduit intérieur (26) et l'ouverture intérieure (231) sont formés par le même alésage ou trou.

5. Contenant souple selon l'une des revendications précédentes, **caractérisé par le fait que** la bride (21) du au moins un orifice (2) est agencée dans un renfoncement (114) sur le côté intérieur (111) de la paroi (11).

6. Contenant souple selon l'une des revendications précédentes, **caractérisé par le fait que** la forme de la plaque de base (22) est telle que la plaque de base (22) et la zone adjacente (111a) du côté intérieur de la paroi (11) forment une surface essentiellement plate et lisse.

7. Contenant souple selon l'une des revendications précédentes, **caractérisé par le fait que** le au moins un orifice (2) comprend un autre élément fonctionnel, par exemple un filtre à bulles, un capteur de pression, une membrane de transfert de pression ou un mécanisme de pompage, complet ou en partie.

8. Dispositif pour la libération automatisée d'un médicament liquide, en particulier un dispositif de pompe à perfusion, **caractérisé par le fait qu'**il comprend et/ou incorpore au moins un contenant souple (1) selon l'une quelconque des revendications 1 à 7.
